# NOUVEAU FASCICULE DE BREVET EUROPEEN

(11) **EP 0 799 873 B2**
(45) Date de publication et mention de la décision concernant l'opposition: **27.12.2006**
(45) Mention de la délivrance du brevet: 21.11.2001
(21) Numéro de dépôt: 97400701.5
(22) Date de dépôt: 27.03.1997
(51) Int. Cl.: C09D 183/08, C03C 17/30, C09D 4/00

(54) **Composition pour un revêtement non-mouillable, procédé de traitement d'un vitrage à l'aide de la composition et produits obtenus**
Zusammensetzung für nichtbenetzbare Beschichtung, Verfahren zur Behandlung einer Glasscheibe mittels dieser Zusammensetzung und die erhaltenen Gegenstände
Composition for a non-wettable coating, process for treating glazing pane with this composition and the products obtained

(30) Priorité: 02.04.1996 FR 9604095; 06.06.1996 FR 9606972
(43) Date de publication de la demande: 08.10.1997
(73) Titulaire: SAINT-GOBAIN GLASS FRANCE, 92400 Courbevoie (FR)
(72) Inventeur: Azzopardi, Marie-José, 75009 Paris (FR); Talpaert, Xavier, 75020 Paris (FR); Gauthier, Fabienne, 45600 Saint Père sur Loire (FR)
(74) Mandataire: Muller, René

(56) Documents cités:
- EP-A- 0 513 727
- EP-A- 0 548 775
- EP-A- 0 657 393
- EP-A- 0 738 771
- WO-A-96/06895
- FR-A- 2 674 862
- US-A- 5 266 222
- Huels-Silicon Compounds, Register and Review, 1991
- Plueddemann, Silane Coupling Agents, 2nd Ed., Plenum Press, New York, 1991, pp V, 4,5,16,17

## Description

La présente invention concerne un procédé de fabrication d'un vitrage muni d'un revêtement non mouillable et le vitrage obtenu.

Les vitrages envisagés selon l'invention sont des vitrages en verre minéral et/ou organique. Ils sont utilisés, en particulier, dans le domaine aéronautique, ferroviaire ou automobile. Ils peuvent aussi être utilisés dans le domaine du bâtiment ou dans le domaine de l'aménagement intérieur comme, par exemple, des panneaux décoratifs, pour l'ameublement, etc. Le substrat sur lequel la composition dé l'invention est susceptible d'être appliquée peut d'autre part être constitué par tout matériau comportant des groupes hydroxyles en surface, tel que des produits verriers revêtus ou non de couches minérales et/ou inorganiques, céramiques, vitrocéramiques - par exemple des plaques chauffantes -, des produits vitrifiés, du béton, des dalles. Les applications envisageables de l'invention sont donc à rechercher dans les domaines aussi différents que ceux du vitrage, de l'électroménager, du bâtiment (fenêtres), des ustensiles de cuisine, du mobilier sanitaire (lavabo, baignoire), des matériaux de construction, etc.

Le caractère de mouillabilité auquel il est fait référence vise le fait que des liquides polaires ou non polaires adhérent sur le substrat et forment un film gênant. Par mouillabilité, on désigne aussi la tendance des substrats à retenir le givre, ainsi que les poussières ou salissures de toutes natures, traces de doigts, calcaire, insectes, etc.

La présence d'eau, de givre et/ou de salissures entraîne un aspect inesthétique, une éventuelle diminution de la transparence du substrat ainsi qu'une altération de la vision à travers celui-ci. Ces dernières sont particulièrement gênantes lorsque le substrat est un vitrage utilisé dans le domaine du transport.

On connaît différents types de revêtements non mouillables. Les demandes de brevets EP 492 417, EP 492 545 et EP 672 779 divulguent en particulier une couche non mouillable obtenue à partir d'organosilanes fluorés. Selon ces documents, cette couche est obtenue en appliquant sur la surface d'un substrat une solution contenant des organosilanes fluorés dans un solvant organique non aqueux. Comme solvant organique non aqueux, le document EP 492 545 cite, en particulier, du n-hexadécane, du toluène, du xylène, etc. Ces solvants sont particulièrement appropriés pour un chlorosilane fluoré. Il est également possible, selon ce document, d'utiliser un alcool méthylique ou éthylique comme solvant lorsque le silane fluoré est un alkoxysilane fluoré. Cependant, il est nécessaire de procéder au dépôt de la couche à l'abri de l'humidité, ce qui pose des problèmes pratiques de mise en oeuvre.

L'invention a pour but de procurer une composition pour un revêtement dont les propriétés de mouillabilité soient satisfaisantes et dont le procédé de dépôt soit particulièrement simple et pratique. La composition de l'invention peut, en particulier, être déposée à la surface d'un substrat en présence d'humidité et notamment à l'atmosphère environnante sans restriction d'humidité.

L'invention concerne un procédé selon la revendication 1 utilisant une composition hydrophobe et oléophobe. Cette composition consistant essentiellement selon l'invention, en au moins un alkoxysilane fluoré dont les fonctions alkoxy sont directement liées à l'atome de silicium, un système de solvant(s) aqueux comprenant au moins un alcool et de l'eau et au moins un catalyseur choisi parmi un acide et/ou une base de Bronsted, la proportion d'eau par rapport à l'alcool étant comprise entre 3 et 20 % en volume.

En plus des effets hydrophobe, oléophobe, anti-pluie, anti-givre, anti-salissure, anti-calcaire, etc. procurés par cette composition, d'autres avantages sont obtenus en cas de couches ou empilements fonctionnels sous-jacents. Ce cas fait notamment référence à la demande EP-A1-0 692 463 décrivant un vitrage comprenant un substrat en verre, un empilement fonctionnel, notamment antireflet, bas-émissif et/ou conducteur, recouvert par une couche hydrophobe et oléophobe.

Dans une telle configuration, la composition utilisée selon l'invention protège l'empilement fonctionnel vis-à-vis des conditions climatiques, ou d'une éventuelle attaque chimique, hydrolytique se traduisant, dans ce dernier cas, par une amélioration de la tenue aux atmosphères chaudes et humides et par des résultats supérieurs dans les essais au brouillard salin neutre. On obtient donc une augmentation de la durabilité, voire une quasi-permanence garantie de la fonction antireflet, ou bas-émissive sous-jacente.

De plus, la propriété anti-adhérente du revêtement hydrophobe et oléophobe selon l'invention est particulièrement appréciable lorsqu'il recouvre une couche ou un empilement antireflet, car ces derniers se caractérisent au contraire par la persistance de toutes traces indésirables sur leur surface.

On comprend par solvant aqueux un mélange d'un alcool, avantageusement un alcanol, par exemple un alcanol de faible poids moléculaire comme le méthanol, l'éthanol, le butanol ou l'isopropanol, et d'eau.

Les silanes fluorés utilisés selon l'invention comportent une fonction hydrolysable susceptible de former des silanols de formule Si-OH. La nature de cette fonction influe sur la vitesse d'hydrolyse dudit silane. Elle est choisie de manière à permettre le dépôt de la composition sur le substrat à l'atmosphère environnante. Il s'agit d'une fonction alkoxy.

Les groupements fluor de l'alkoxysilane confèrent à la couche obtenue une hydrophobie et une oléophobie particulièrement marquées. II confère, en outre, une bonne résistance aux rayonnements ultraviolets. Les alkoxysilanes fluorés utilisés selon l'invention sont avantageusement des perfluoroalkoxysilanes de formule : 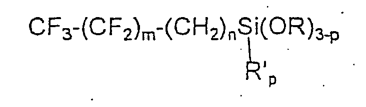 avec :
- m = 0 à 15
- n=1 à 5
- p = 0, 1, 2
- R = alkyl
- R' = alkyl ou H

La longueur de la chaîne carbonée de l'organosilane est, de préférence, relativement longue. Avantageusement, le nombre de carbones fluorés est plus important que le nombre de carbones alkyls afin de conférer une densité plus importante en fluor vers l'extérieur: m est avantageusement au moins le double de n.

L'alkoxysilane fluoré utilisé de façon préférée selon l'invention est choisi parmi les perfluorotrialkoxysilanes suivants, seuls ou en mélange:
CF₃-(CF₂)₅-(CH₂)₂Si(OR)₃ ; CF₃-(CF₂)₇-(CH₂)₂Si(OR)₃; CF₃-(CF₂)₉-(CH₂)₂Si(OR)₃
- R est un alkyl, de préférence un méthyl ou un éthyl ; il peut également être choisi parmi les perfluorodialkoxysilanes suivants, seuls ou en mélange :
   CF₃-(CF₂)₅-(CH₂)₂SiH(OR)₂; CF₃-(CF₂)₇-(CH₂)₂SiH(OR)₂; CF₃-(CF₂)₉-(CH₂)₂SiH(OR)₂
- R est un alkyl, de préférence un méthyl ou un éthyl.

La proportion d'alkoxysilane fluoré est comprise entre 0,05 et 5 % en poids par rapport à la composition et, de préférence, est comprise entre 1 et 3 % en poids.

Les proportions des différents constituants de la composition influent sur le caractère de mouillabilité du revêtement obtenu. La proportion d'eau, par rapport à l'alcool, est comprise entre 3 et 20 % en volume et, de préférence, est de l'ordre de 10 % en volume. Ainsi, sans que l'invention soit liée à ce type d'interprétation, les alkoxysilanes présents dans un système de solvant(s) aqueux peuvent commencer à s'hydrolyser pour former des silanols susceptibles de réagir avec les groupes réactifs à la surface du substrat. Or, une proportion trop importante de silanes hydrolysés peut conduire à une homopolymérisation. Le nombre de silanols susceptibles de réagir avec la surface du substrat est alors diminué. De même, une proportion trop faible de silanes hydrolysés peut conduire à un nombre trop faible de silanes fixés à la surface du substrat.

Le catalyseur selon l'invention permet de catalyser la réaction d'hydrolyse. Il est choisi parmi un acide et/ou une base de Bronsted, c'est-à-dire qu'il est susceptible de libérer un ion H⁺ ou un ion OH-. Il s'agit par exemple d'un acide tel que l'acide chlorhydrique ou acétique. Sa proportion influe également sur le caractère de mouillabilité du revêtement. Elle est comprise entre 0,005 et 20 % en poids par rapport à la composition et est, de préférence, de l'ordre de 10 % en poids par rapport à la composition.

Le revêtement est obtenu par réaction des alkoxysilanes hydrolysés avec les groupes réactifs de la surface du substrat. C'est une liaison de type covalent. La structure générale de la couche est alors globalement, pour un organosilane, une liaison covalente au point de fixation sur la surface du substrat et une ou deux liaisons covalentes avec des molécules d'organosilanes voisines par les autres fonctions hydrolysables.

L'épaisseur de la couche obtenue est comprise entre 10 et 150 Angströms et, de préférence, entre 10 et 100 Angströms. Il s'agit d'une couche transparente, n'altérant pas la transparence et la vision à travers le substrat.

La composition utilisée selon l'invention est appliquée sur au moins une partie de la surface d'un substrat comprenant des groupes notamment OH susceptibles de réagir avec le silane hydrolysé de la composition. Le substrat peut être un verre minéral, une matière plastique, telle que par exemple du polycarbonate, ou un support revêtu d'au moins une couche minérale et/ou inorganique.

Comme couches, on peut citer, par exemple, des couches fonctionnelles telles que des couches anti-rayures, anti-abrasion, anti-reflets, des couches décoratives, bas-émissives comme mentionné précédemment.

La compositions selon l'invention peut aussi être déposée sur une couche au moins partiellement dégradée. Cette dégradation peut être, par exemple, due au vieillissement naturel ou à une abrasion mécanique ou chimique. Il peut s'agir d'une abrasion due aux frottements des essuie-glace ou à l'impact de la pluie, de la grêle, de chocs. La couche régénérée sur une couche dégradée étant aussi performante que la couche initiale, il n'est pas nécessaire de procéder à une préparation de surface telle qu'une abrasion, un polissage avant de procéder au dépôt de la composition de l'invention.

Néanmoins, la durabilité de la couche selon l'invention est améliorée par un traitement préalable du substrat à l'aide d'une composition de primage contenant un silane du type SiX₄, avec X étant une fonction hydrolysable par exemple chlorée ou alkoxy.

En effet, un primage augmente la réactivité du verre, qui a pour conséquence une amélioration de l'accrochage du silane fluoré. De plus, le primage désorganise la couche fluorée, et permet donc de la former en épaisseurs plus importantes, au moins égales à 100 Angströms, sans toutefois excéder 500 Angströms : il ne s'agit pas de couche monomoléculaires. L'augmentation de la quantité de fluor déposé ainsi obtenue se traduit par une durabilité accrue dans des conditions d'exposition au rayonnement ultraviolet.

D'autre part, aux valeurs susmentionnées de l'épaisseur de la couche fluorée, une rayure de celle-ci n'est pas visible à l'oeil nu.

L'invention concerne également le procédé de fabrication d'un vitrage muni d'un revêtement hydrophobe et oléophobe.

Le procédé de fabrication du vitrage comprend plus précisément les étapes suivantes :
- fabrication de la composition susceptible de constituer la couche hydrophobe et oléophobe,
- fabrication de la composition de primage,
- dépôt de ladite composition sur au moins une partie de la surface du vitrage qui a été traitée par la composition de primage.

La fabrication de la composition comprend notamment le mélange de l'alkoxysilane fluoré de l'invention avec un système solvant aqueux et au moins un catalyseur. Ce mélange peut se faire en présence d'humidité et notamment à l'atmosphère environnante. Le produit obtenu est une solution réactive par exemple pendant 24 heures. Cette solution est déposée sur au moins une partie de la surface du vitrage, de préférence entre 10 minutes et une heure après sa fabrication. Le dépôt s'effectue par mise en contact du vitrage avec la solution. Cette mise en contact peut se faire par tout moyen, par exemple pa rcoulée, pulvérisation, centrifugation, immersion, trempé, au moyen d'un rouleau enducteur ou d'une brosse ou, de préférence, par chiffonnage. Le substrat peut être à température ambiante ou chauffé à une température inférieure à 300°C. Au-delà de 300°C, la couche risque de se dégrader.

Le traitement de primage peut être réalisé par la mise en oeuvre du même procédé de dépôt que celui utilisé pour le dépôt de la couche hydrophobe et oléophobe et en utilisant le même système de solvant aqueux et de catalyseur. La composition de primage peut contenir de 0,001 à 5 % en poids de SiX₄. Le traitement par la composition de primage a pour effet d'augmenter le nombre de sites réactifs (sites hydroxyles) du substrat.

La surface du vitrage à enduire doit être propre. La propreté du vitrage détermine le nombre de sites réactifs du substrat susceptibles de réagir avec les groupements alkoxysilanes hydrolysés. Elle a pour but d'éviter la présence de toutes les contaminations, essentiellement organiques, adsorbées à la surface du substrat et susceptibles de s'opposer à la réaction des alkoxysilanes hydrolysés sur les sites réactifs du substrat. La surface du vitrage est préalablement nettoyée, par exemple à l'aide d'un tensioactif.

Les substrats préparés avec les couches de l'invention sont à la fois hydrophobes et oléophobes. Ils résistent bien aux rayonnements ultraviolets, aux agressions chimiques et à l'abrasion mécanique. Ils sont avantageusement utilisés comme substrats anti-pluie, anti-givre, anti-salissures, anti-contaminants, anti-calcaire, ... Ils sont particulièrement utiles comme vitrages dans des véhicules terrestres, aéronautiques ou pour des bâtiments.

Les exemples suivants, non limitatifs, illustrent les caractéristiques et avantages de l'invention :
- l'exemple 1 illustre le caractère non mouillable de la couche selon l'invention,
- l'exemple 2 est un exemple comparatif de l'exemple 1,
- l'exemple 3 illustre la résistance à l'abrasion d'une variante de l'invention,
- l'exemple 4 est un exemple comparatif de l'exemple 3,
- les exemples 5a et 5b illustrent un traitement préalable du substrat par une composition de primage.

La résistance à l'abrasion de la couche est mesurée par le test dit « d'essuie-glace ». Les échantillons sont soumis à l'action d'un balai d'environ 50 cm de long et exerçant une force de 45 Newtons sur l'échantillon. Après un nombre donné d'allers et de retours du balai, l'angle de contact d'une goutte d'eau est mesuré.

### EXEMPLE 1 (hors invention)

Quatre échantillons de verre flotté silico-sodo-calcique sont nettoyés.

Chaque échantillon est traité au moyen d'une solution contenant un mélange de perfluorotrialkoxysilane de formule :
CF₃-(CF₂)₅-(CH₂)₂Si(OC₂H₅)₃ ; CF₃-(CF₂)₇-(CH₂)₂Si(OC₂H₅)₃ ; CF₃-(CF₂)₉-(CH₂)₂Si(OC₂H₅)₃
dans un système de solvants aqueux comprenant de l'éthanol, un catalyseur, en l'occurrence de l'acide acétique, et de l'eau. La solution est appliquée par chiffonnage. Seules les proportions de ces différents constituants varient selon les échantillons. De l'éthanol à 95 % est utilisé. Dans le tableau suivant, les proportions d'alcool à 95 % ont été converties en pourcentages correspondants d'alcool absolu et d'eau. L'hydrophobie de la couche ainsi constituée est quantifiée en mesurant l'angle de contact θ d'une goutte d'eau sur ladite couche.

Les résultats sont les suivants:

| Echantillon | % éthanol absolu | % eau | % acide acétique | % silane | θ eau |
|---|---|---|---|---|---|
| 1 | 85,5 | 4,5 | 10 | 0,1 | 90° |
| 2 | 85,5 | 4,5 | 10 | 3 | 105 à 110° |
| 3 | 76 | 14 | 10 | 0,1 | 100 à 105° |
| 4 | 76 | 14 | 10 | 3 | 105 à 110° |

On considère que le caractère non mouillable de la couche est satisfaisant lorsque l'angle de contact initial d'une goutte d'eau sur ladite couche est supérieur à 90°.

Cet exemple illustre le caractère hydrophobe de la couche obtenue.

### EXEMPLE 2 - EXEMPLE COMPARATIF

Cet exemple est un exemple comparatif de l'exemple 1.

Quatre échantillons sont traités de la même manière que dans l'exemple 1 excepté que les compositions utilisées ne comprennent pas de catalyseur. Les proportions des constituants du système de solvant(s) varient également. L'éthanol utilisé est de l'éthanol à 95 %. Dans le tableau ci-dessous, les proportions d'alcool à 95 % ont été converties en pourcentages correspondant d'alcool absolu et d'eau.

Les résultats sont les suivants :

| Echantillon | % éthanol absolu | % eau | % silane | θ eau |
|---|---|---|---|---|
| 1 | 95 | 5 | 0,1 | 61 à 70° |
| 2 | 95 | 5 | 3 | 56 à 80° |
| 3 | 85,5 | 14,5 | 0,1 | 40 à 70° |
| 4 | 85,5 | 14,5 | 3 | 70 à 80° |

Le caractère non mouillable des couches obtenues ne donne pas entièrement satisfaction : les angles de contact d'une goutte d'eau sur la couche sont inférieurs à 90°.

### EXEMPLE 3 (hors invention)

Un échantillon de verre flotté de type silico-sodo-calcique subit quatre traitements successifs avec une solution identique à celle utilisée pour traiter l'échantillon n° 4 de l'exemple 1. La sous-couche sur laquelle est déposée par chiffonnage la composition de l'invention est, à chaque fois, préalablement dégradée par le test dit « d'essuie-glace ».

Les résultats sont les suivants :

| | Mouillabilité de la sous-couche | | Mouillabilité de la couche obtenue | |
|---|---|---|---|---|
| | θ | Nombre de cycles d'essuie-glace utilisés pour dégrader la couche | θ initial | θ après le test d'essuie-glace |
| 1 er traitement | - | - | 105-110° | 60° (50.000 cycles) |
| 2ème traitement | 60° | 50.000 | 100-105° | 65° (70.000 cycles) |
| 3éme traitement | 65° | 70.000 | 110° | 70° (100.000 cycles) |
| 4éme traitement | 70° | 100.000 | 115° | 70° (100.000 cycles) |

On considère que la résistance à l'abrasion de la couche est satisfaisante si l'angle de contact d'une goutte d'eau sur la couche est supérieur à 60° après le test d'essuie-glace.

Cet exemple illustre une résistance améliorée de la couche obtenue sur une sous-couche au moins partiellement dégradée.

Les régénérations sur des couches précédemment dégradées au test d'essuie-glace avion présentent des tenues au moins équivalentes et, dans certains cas, supérieures à celles de la couche initiale. On a initialement θ eau 60° après 50.000 cycles d'essuie-glace avion. Après 3 ou 4 cycles d'abrasion à l'essuie-glace avion et de réapplication de la couche anti-pluie, on atteint θ eau = 60° après 100.000 cycles d'essuie-glace avion.

### EXEMPLE 4 - EXEMPLE COMPARATIF

Cet exemple est un exemple comparatif de l'exemple 3. Un échantillon de verre flotté de type silico-sodo-calcique subit deux traitements à l'aide d'une solution contenant 0,5 % en volume d'un chlorosilane fluoré de formule : CF₃-(CF₂)₇-(CH₂)₂SiCl₃ dans un solvant non aqueux tel que le décane. Comme dans l'exemple 3, la première couche obtenue par le premier traitement est dégradée à l'aide du test dit « d'essuie-glace » avant de déposer la deuxième couche.

Les résultats sont les suivants :

| | Mouillabilité de la sous-couche | | Mouillabilité de la couche obtenue | |
|---|---|---|---|---|
| | θ | Nombre de cycles d'essuie-glace utilisés pour dégrader la couche | θ initial | θ après le test d'essuie-glace |
| 1er traitement | - | - | 100-105° | 70° (50.000 cycles) |
| 2ème traitement | 65-80° | 50.000 | 95-110° | 55° (50.000 cycles) |

Cet exemple illustre la mauvaise résistance à l'abrasion d'une couche de l'art antérieur déposée sur une sous-couche au moins partiellement dégradée : l'angle de contact d'une goutte d'eau sur la couche obtenue ayant subi 50.000 cycles d'essuie-glace est inférieur à 60° en moyenne.

### EXEMPLES 5a ET 5b

On opère comme dans le cas de l'échantillon 4 de l'exemple 1 sauf que l'on traite le substrat au préalable par une composition de primage contenant 76 parties d'éthanol absolu, 14 parties d'eau, 10 parties d'acide acétique et 1 partie de silane de type SiX₄, à savoir une partie de Si(OCH₃)₄ pour l'exemple 5a et une partie de Si(OC₂H₅)₄ pour l'exemple 5b.

Les résultats sont les suivants :

| Exemple | θ initial | θ après le test d'essuie-glace |
|---|---|---|
| 5a | 105° | 100° après 250.000 cycles |
| 5b | 110° | 90° après 100.000 cycles |

Comparés au premier traitement du tableau de l'exemple 3, on note une amélioration significative de la tenue de la couche hydrophobe et oléophobe au test d'essuie-glace.

## Revendications

1. Procédé de fabrication d'un vitrage muni, sur au moins une partie de sa surface, d'une couche hydrophobe et oléophobe, **caractérisé en ce qu'**il comprend notamment les étapes suivantes :
- préparation d'une composition de primage obtenue à partir d'un mélange d'un silane SiX₄, X étant une fonction hydrolysable, avec un système de solvant(s) aqueux et au moins un catalyseur choisi parmi un acide et/ou une base de Bronsted, la teneur en SiX₄ étant comprise entre 0,001 et 5% en poids de la composition,
- préparation d'une composition obtenue à partir d'un mélange d'un alkoxysilane fluoré de formule :
CF₃-(CF₂)ₘ-(CH₂)ₙ(OR)₃₋ₚ (R'ₚ)
avec :
• m = 0 à 1 5
• n = 1 à 5
• p = 0, 1, 2
• R = alkyls
• R' = alkyl ou H,
avec un système de sotvant(s) aqueux comprenant au moins un alcool et de l'eau et au moins un catalyseur choisi parmi un acide et/ou une base de Bronsted, la proportion d'eau par rapport à l'alcool étant comprise entre 3 et 20% en volume.
- traitement d'au moins la partie de la surface du vitrage destinée à recevoir la couche hydrophobe et oléophobe, au préalable, avec ladite composition de primage,
- dépôt de la composition obtenue à partir du mélange de l'alkoxysilane fluoré avec le système de solvant(s) aqueux sur la partie de la surface du vitrage préalablement traité avec ladite composition de primage.

2. Procédé selon la revendication 1, **caractérisé en ce que** l'alcool est un alcool choisi parmi le méthanol, l'éthanol, le butanol et l'isopropanol.

3. Procédé selon la revendication 1 ou 2, **caractérisé en ce que** la proportion d'eau par rapport à l'alcool dans le système de solvant(s) aqueux est de l'ordre de 10% en volume.

4. Procédé selon l'une des revendications 1 à 3, **caractérisé en ce que** le dépôt de la composition obtenue à partir du mélange de l'alkoxysilane fluoré avec le système de solvant(s) aqueux se fait par chiffonnage.

5. Procédé selon l'une des revendications 1 à 4, **caractérisé en ce que** la composition obtenue à partir du mélange de l'alkoxysilane fluoré avec le système de solvant(s) aqueux est déposée au maximum 24 heures, de préférence entre 10 minutes et 1 heure, après sa préparation.

6. Vitrage obtenu par le procédé selon l'une des revendications 1 à 5.

7. Vitrage selon la revendication 6, comprenant au moins une couche ou un empilement fonctionnel(le), recouvert(e) par une couche hydrophobe et oléophobe.

## Claims

1. Process for manufacturing a glazing pane provided, on at least one part of its surface, with a hydrophobic and oleophobic layer, **characterized in that** it comprises especially the following steps:
- preparation of a priming composition obtained from a mixture of a silane SiX₄, X being a hydrolysable group, with an aqueous solvent system and at least one catalyst chosen from a Brønsted acid and/or base, the SiX₄ content being between 0.001 and 5% by weight of the composition;
- preparation of a composition obtained from a mixture of a fluorinated alkoxysilane of formula:
CF₃-(CF₂)ₘ-(CH₂)ₙSi(OR)₃₋ₚ(R'ₚ)
with:
• m = 0 to 15;
• n = 1 to 5;
• p = 0, 1 or 2;
• R = an alkyl group;
• R' = an alkyl group or H,
with an aqueous solvent system comprising at least one alcohol and water and at least one catalyst chosen from a Brønsted acid and/or base, the proportion of water with respect to alcohol being between 3 and 20% by volume;
- prior treatment of at least that part of the glazing pane surface intended to receive the hydrophobic and oleophobic layer with said priming composition; and
- deposition of the composition obtained from the mixture of the fluorinated alkoxysilane with the aqueous solvent system on that part of the glazing pane surface pretreated with said priming composition.

2. Process according to Claim 1, **characterized in that** the alcohol is an alcohol chosen from methanol, ethanol, butanol and isopropanol.

3. Process according to Claim 1 or 2, **characterized in that** the proportion of water with respect to alcohol in the aqueous solvent system is around 10% by volume.

4. Process according to one of Claims 1 to 3, **characterized in that** deposition of the composition obtained from the mixture of the fluorinated alkoxysilane with the aqueous solvent system is carried out by wiping-on.

5. Process according to one of Claims 1 to 4, **characterized in that** the composition obtained from the mixture of the fluorinated alkoxysilane with the aqueous solvent system is deposited at a maximum of 24 hours, preferably between 10 minutes and 1 hour, after its preparation.

6. Glazing pane obtained by the process according to one of Claims 1 to 5.

7. Glazing pane according to Claim 6, comprising at least one functional layer or multilayer, covered by a hydrophobic and oleophobic layer.

## Patentansprüche

1. Verfahren zur Herstellung einer Glasscheibe, die auf wenigstens einem Teil ihrer Oberfläche mit einer hydrophoben und oleophoben Schicht versehen ist, **dadurch gekennzeichnet, dass** es insbesondere die Stufen:
- Herstellung einer Haftvermittlungszusammensetzung, die aus einem Gemisch aus einem Silan, SiX₄, wobei X eine hydrolysierbare Funktion bedeutet, mit einem System aus wässrigem/wässrigen Lösungsmittel/n und mindestens einem Katalysator, der aus einer Bronsted-Säure und/oder -Base ausgewählt ist, erhalten wird, wobei der SiX₄-Gehalt 0,001 bis 5 Gew.-% der Zusammensetzung beträgt,
- Herstellung einer Zusammensetzung, die aus einem Gemisch aus einem fluorierten Alkoxysilan mit der Formel
CF₃-(CF₂)ₘ-(CH₂)ₙSi(OR)₃₋ₚ(R'ₚ),
wobei:
- m = 0 bis 15,
- n = 1 bis 5,
- p =0, 1, 2,
- R = Alkyle und
- R' = Alkyl oder H
bedeutet,
mit einem System aus wässrigem/wässrigen Lösungsmittel/n, das mindestens einen Alkohol und Wasser umfasst, und mindestens einem Katalysator, der aus einer Brønsted-Säure und/oder -Base ausgewählt ist, wobei der Wasseranteil in Bezug auf den Alkohol 3 bis 20 Vol.-% beträgt, erhalten wird,
- Vorbehandlung wenigstens des Teils der Oberfläche der Glasscheibe, der vorgesehen ist, die hydrophobe und oleophobe Schicht aufzunehmen, mit der Haftvermittlungszusammensetzung und
- Aufbringen der Zusammensetzung, die aus dem Gemisch aus dem fluorierten Alkoxysilan mit dem System aus wässrigem/wässrigen Lösungsmittel/n erhalten worden ist, auf den Teil der Oberfläche der Glasscheibe, der mit der Haftvermittlungszusammensetzung vorbehandelt worden ist,
umfasst.

2. Verfahren nach Anspruch 1, **dadurch gekennzeichnet, dass** der Alkohol aus Methanol, Ethanol, Butanol und Isopropanol ausgewählt ist.

3. Verfahren nach Anspruch 1 oder 2, **dadurch gekennzeichnet, dass** der auf den Alkohol bezogene Wasseranteil im System aus wässrigem/wässrigen Lösungsmittel/n etwa 10 Vol.-% beträgt.

4. Verfahren nach einem der Ansprüche 1 bis 3, **dadurch gekennzeichnet, dass** die Zusammensetzung, die aus dem Gemisch aus fluoriertem Alkoxysilan mit dem System aus wässrigem/wässrigen Lösungsmittel/n erhalten worden ist, mit einem Lappen aufgebracht wird.

5. Verfahren nach einem der Ansprüche 1 bis 4, **dadurch gekennzeichnet, dass** die Zusammensetzung, die aus dem Gemisch aus fluoriertem Alkoxysilan mit dem System aus wässrigem/wässrigen Lösungsmittel/n erhalten worden ist, höchstens 24 Stunden und vorzugsweise zwischen 10 Minuten und 1 Stunde nach ihrer Herstellung aufgebracht wird.

6. Glasscheibe, die durch das Verfahren nach einem der Ansprüche 1 bis 5 erhalten worden ist.

7. Glasscheibe nach Anspruch 6, die mindestens eine Funktionsschicht oder einen Funktionsschichtenaufbau umfasst, die/der mit einer hydrophoben und oleophoben Schicht überzogen ist.
